# FASCICULE DE BREVET EUROPEEN

(11) **EP 0 575 232 B1**
(45) Date de publication et mention de la délivrance du brevet: **15.02.1995**
(21) Numéro de dépôt: 93401512.4
(22) Date de dépôt: 11.06.1993
(51) Int. Cl.: A61B 17/14

(54) **Dispositif de coupe rotulienne pour la pose d'une prothèse totale du genou**
Kniescheiberesektionsvorrichtung zum Einsetzen einer Gesamtknieprothese
Patellar cutting apparatus for implanting a total knee prosthesis

(30) Priorité: 16.06.1992 FR 9207290
(43) Date de publication de la demande: 22.12.1993
(73) Titulaire: DEVELOPPEMENT IMPLANTS ORTHOPEDIQUES ET MEDICAUX, F-25460 Etupes (FR); PROTEK SYNTHES, 25460 Etupes (FR)
(72) Inventeur: Aebi, Jürg, F-75116 Paris (FR); Beaufils, Philippe, F-75116 Paris (FR); De Lestang, Michel, F-75116 Paris (FR); Gaffuri, Jean-Gilles, F-75116 Paris (FR); Hourlier, Hervé, F-75116 Paris (FR); Lallement, Jean-Jacques, F-75116 Paris (FR); Legroux, Philippe, F-75116 Paris (FR); Levai, Jean-Paul, F-75116 Paris (FR); Pondaven, Gérard, F-75116 Paris (FR); Schuster, Pierre, F-75116 Paris (FR); Vergnat, Christian, F-75116 Paris (FR); Bouraly, Jean-Pierre, F-75116 Paris (FR)
(74) Mandataire: Lemoyne, Didier

(56) Documents cités:
- EP-A- 0 466 659
- WO-A-91/04715
- US-A- 4 952 213
- US-A- 5 108 401

## Description

La présente invention concerne un dispositif de coupe rotulienne pour la pose d'une prothèse totale de genou.

L'invention trouve une application particulièrement avantageuse dans le domaine des prothèses de resurfaçage des os concernant l'articulation du genou.

Les prothèses totales tricompartimentales de genou sont constituées d'un composant tibial, d'un composant rotulien et d'un composant fémoral présentant notamment une trochlée prothétique. Dans ce type de prothèse, la rotule est destinée à se déplacer dans ladite trochlée prothétique via ledit composant rotulien.

De manière habituelle dans une intervention de pose de prothèse totale de genou, la rotule anatomique est coupée, puis percée de trous d'ancrage pour l'implantation du futur composant rotulien prévu pour venir coopérer avec la trochlée prothétique aménagée sur le composant fémoral. Un tel dispositif est décrit dans le document WO-A-91/04715, document qui se réfère à un davier de coupe de rotule comprenant une palette rotulienne prévue pour être mise en appui coutre la rotule et des moyens de coupe de la rotule.

Actuellement, les coupes rotuliennes sont toujours réalisées séparément du fémur et sans repère autre qu'anatomique.

Il en résulte que, dans certains cas, la coupe n'étant pas parallèle au plan de la trochlée, le composant rotulien est mal posé. La rotule est dite en bascule et a donc souvent tendance à se luxer, c'est-à-dire à sortir de la gorge constituée par la trochlée du composant fémoral. Schématiquement, l'action de la rotule lors de la flexion/extension du genou peut être assimilée à une poulie qui compense la tendance de l'articulation fémoro-tibiale à se déboîter. Dans la mesure où la poulie équivalente est positionnée de travers, celle-ci tend à porter à faux et par suite à sortir du logement dans lequel elle se trouve engagée.

A titre d'exemple, on peut citer un procédé de coupe qui consiste en premier lieu à luxer la rotule, puis à la maintenir dans un davier pour y être coupée ou fraisée. Toutefois, ce type de technique ne permet pas d'obtenir une coupe de la rotule parfaitement parallèle au plan de la trochlée du composant fémoral.

Aussi, le problème technique à résoudre par l'objet de la présente invention est de proposer un dispositif de coupe rotulienne pour la pose d'une prothèse totale de genou, qui permettrait de réaliser une coupe systématiquement parallèle au plan de la trochlée.

La solution au problème technique posé consiste, selon la présente invention en ce que ledit dispositif comporte, d'une part, un composant fémoral d'essai présentant une trochlée prothétique, au moins un orifice latéral aménagé sur au moins un bord dudit composant fémoral d'essai, et un trou central aménagé au centre de ladite trochlée, et, d'autre part, un ancillaire comprenant un téton de fixation destiné à coopérer avec ledit orifice latéral du composant fémoral d'essai, une palette rotulienne prévue pour être mise en appui contre la rotule, un moyen de centrage de ladite palette par rapport audit trou central de la trochlée prothétique, des moyens de déplacement de la palette rotulienne permettant un mouvement transversal à ladite trochlée et un mouvement axial parallèle au trou central, des moyens de blocage en position de ladite palette, et des moyens de coupe de la rotule parallèlement au plan de ladite trochlée.

Le dispositif conforme à l'invention permet donc d'obtenir, grâce à des références fixes prises par rapport au composant fémoral d'essai lui-même, une coupe de la rotule parfaitement parallèle au plan de la trochlée prothétique.

Dans un premier mode de réalisation du dispositif, objet de l'invention, il est prévu que lesdits moyens de coupe sont constitués par une fente latérale parallèle au plan de la trochlée prothétique, aménagée dans un premier gabarit disposé sur l'ancillaire, et dans laquelle est introduite une scie oscillante. Ce mode de réalisation, qui rend possible la coupe rotulienne rotule en place, présente cependant l'inconvénient que le chirurgien ne peut voir exactement de quelle manière la coupe est réellement effectuée.

C'est pourquoi l'invention prévoit un deuxième mode de réalisation du dispositif de coupe rotulienne qui offre au praticien la possibilité de surveiller la coupe de la rotule au fur et à mesure de son exécution. Ce deuxième mode de réalisation est remarquable en ce que lesdits moyens de coupe sont constitués par une platine de coupe engagée sur deux broches traversant la rotule et mises en place parallèlement au plan de la trochlée prothétique à partir de deux trous de passage aménagés dans un second gabarit disposé sur l'ancillaire.

La description qui va suivre en regard des dessins annexés, donnés à titre d'exemples non limitatifs, fera bien comprendre en quoi consiste l'invention et comment elle peut être réalisée.

La Figure 1a est une vue de face d'un premier dispositif de coupe rotulienne conforme à l'invention.

La Figure 1b est une vue de côté correspondant à la vue de face de la Figure 1a.

La Figure 2a est une vue de face d'un deuxième dispositif de coupe rotulienne conforme à l'invention.

La Figure 2b est une vue de côté correspondant à la vue de face de la Figure 2a.

La Figure 3a est une vue de côté d'une rotule munie d'une platine de coupe.

La Figure 3b est une vue de face correspondant à la vue de côté de la Figure 3a.

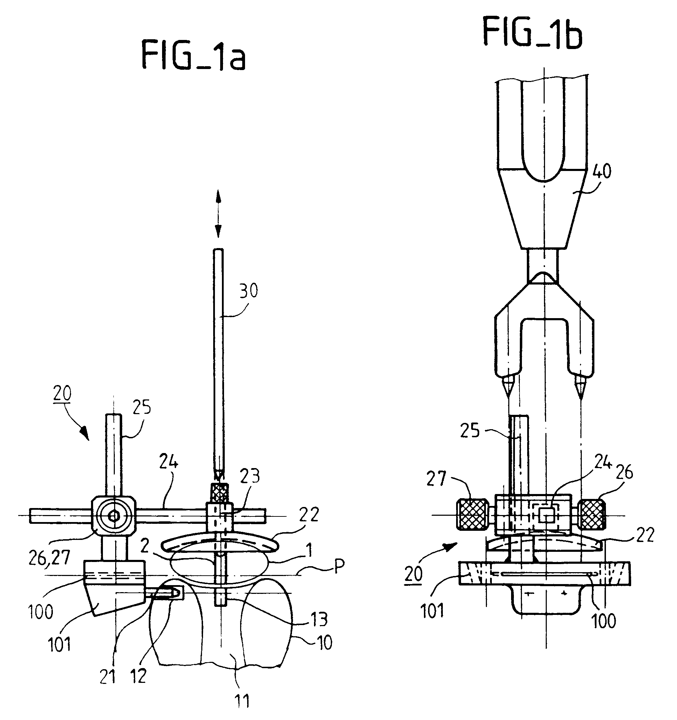

Les Figures 1a et 1b illustrent en vues de face et de côté un premier dispositif pour la pose d'une prothèse totale de genou comportant, d'une part, un composant fémoral 10 d'essai présentant une trochlée prothétique 11, au moins un orifice latéral 12 aménagé sur au moins un bord dudit composant fémoral d'essai, et un trou central 13 aménagé au centre de ladite trochlée. D'autre part, le dispositif de coupe rotulienne montré aux Figures 1a et 1b comporte également un ancillaire 20 comprenant un téton 21 de fixation destiné à coopérer avec l'orifice latéral 12 du composant fémoral 10 d'essai, de manière à fixer l'ancillaire sur ledit composant fémoral pendant toute la durée de l'intervention. De façon pratique, il est prévu deux tétons de fixation et deux orifices latéraux correspondants sur chaque bord du composant fémoral d'essai. L'ancillaire 20 des Figures 1a et 1b comprend, en outre, une palette rotulienne 22 en forme de dôme, prévue pour être mise en appui contre la rotule 1.

La palette rotulienne 22 est munie d'un moyen de centrage par rapport au trou central 13 de la trochlée prothétique, constitué par un trou 23 de centrage aménagé au centre de la palette et destiné à être amené en coïncidence avec ledit trou central 13 à l'aide d'une broche 30 d'alignement et par la mise en oeuvre d'un mouvement transversal à la trochlée 11 autorisé par des moyens de déplacement de la palette 22. Dans le mode de réalisation montré aux Figures 1a et 1b, lesdits moyens de déplacement sont constitués par une tige transversale 24 supportant ladite palette rotulienne, coulissante dans ledit mouvement transversal, et par une tige axiale 25 le long de laquelle coulisse ladite tige transversale dans un mouvement de déplacement axial parallèlement au trou central 13 de la trochlée prothétique 11.

On notera également sur les Figures 1a et 1b la présence sur l'ancillaire 20 de moyens de blocage en position de la palette rotulienne 22, formés par une première molette 26 de blocage de la palette sur la tige transversale 24 et par une deuxième molette 27 de blocage de la tige transversale sur la tige axiale 25.

Enfin, l'ancillaire de coupe rotulienne des Figures 1a et 1b comporte des moyens de coupe de la rotule 1 parallèlement au plan P de la trochlée prothétique 11, constitués, dans cet exemple, par une fente latérale 100 parallèle au plan P de la trochlée, aménagée dans un premier gabarit 101 disposé sur l'ancillaire 20 et dans laquelle est introduite une scie oscillante non représentée sur les Figures 1a et 1b.

Le procédé de coupe rotulienne mettant en oeuvre le dispositif illustré aux Figures 1a et 1b comprend alors les opérations suivantes :
- luxer la rotule 1,
- mettre en place le composant fémoral 10 d'essai,
- fixer audit composant fémoral d'essai l'ancillaire 20 rotulien de coupe par coopération des tétons 21 de fixation avec les orifices latéraux 12,
- amener, par la mise en oeuvre du mouvement transversal desdits moyens 24, 25 de déplacement, le trou 23 de centrage de la palette rotulienne 22 en regard du trou central 13 du composant fémoral d'essai,
- introduire la broche 30 d'alignement à travers le trou 23 de centrage jusque dans le trou central 13,
- bloquer la palette rotulienne 22 en position centrée par rapport à la trochlée 11 au moyen des vis 26, 27 de blocage,
- retirer la broche 30 d'alignement,
- dégager la palette 22 par la mise en oeuvre du mouvement axial des moyens 24, 25 de déplacement,
- réduire et plaquer la rotule 1 sur le composant fémoral 10 d'essai,
- appliquer la palette rotulienne 22 contre la rotule de manière à la centrer par rapport à la trochlée 11,
- percer la rotule 1 d'un trou traversant axial 2 à l'aide d'un moyen de perçage engagé dans le trou 23 de centrage de la palette rotulienne,
- introduire, à travers le trou de centrage, une broche à butée dans ledit trou traversant axial 2,
- couper la rotule à l'aide de la scie oscillante à travers la fente latérale 100.

Durant l'opération de coupe, la rotule 1 peut être maintenue fermement en position au moyen d'un manche 40 d'appui appliqué contre la palette rotulienne 22, ainsi que le montre la Figure 1b.

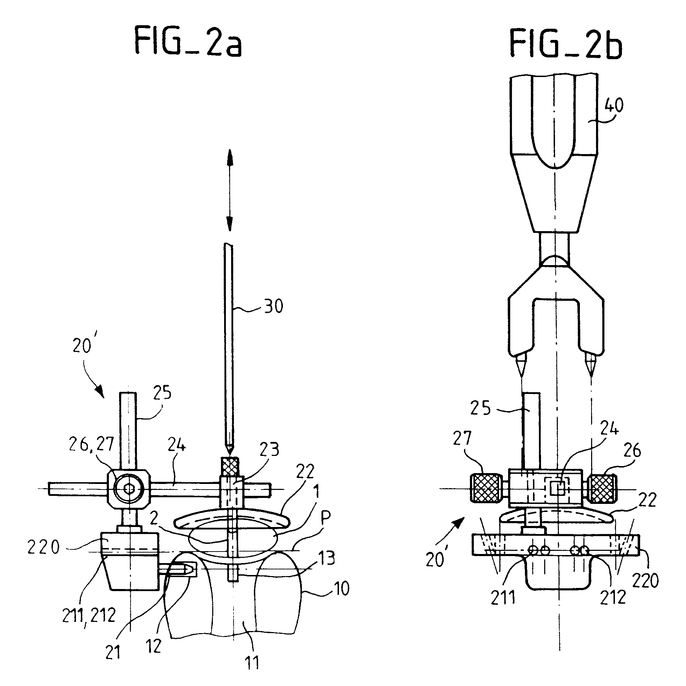

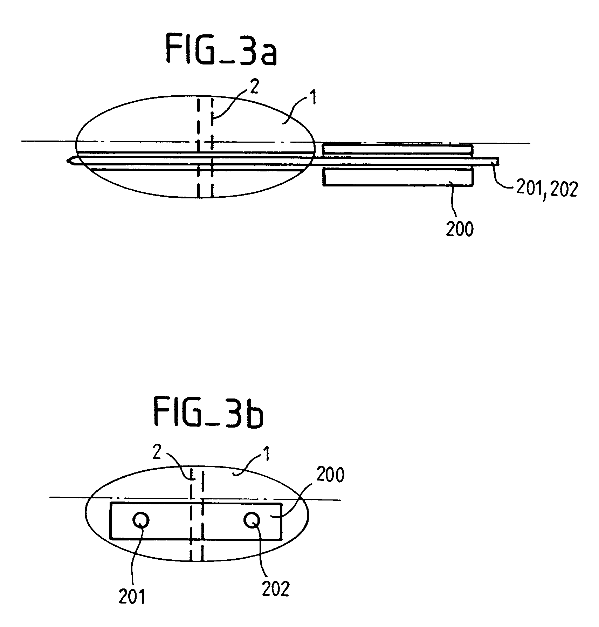

Les Figures 2a, 2b, 3a et 3b illustrent un deuxième dispositif de coupe rotulienne qui comporte des éléments communs avec le dispositif des Figures 1a et 1b, ces éléments communs portant les mêmes numéros de référence. Le dispositif des Figures 2a, 2b, 3a et 3b se distingue du dispositif précédemment décrit en ce que les moyens de coupe sont constitués par une platine 200 de coupe engagée sur deux broches 201, 202 traversant la rotule 1 et mise en place parallèlement au plan de la trochlée prothétique 11 à partir de deux trous 211, 212 de passage aménagés dans un second gabarit 220 disposé sur l'ancillaire 20'.

La Figure 1b montre deux jeux de trous de passage entre lesquels le praticien peut choisir en fonction de la taille de la rotule anatomique considérée.

De même, on peut voir sur les Figures 3a et 3b que la platine 200 de coupe est dissymétrique de manière à présenter deux niveaux de coupe possibles.

Le procédé de coupe rotulienne utilisant ce deuxième dispositif comporte les mêmes premières étapes que le procédé mettant en oeuvre le premier dispositif de coupe, jusqu'à l'étape d'introduction de la broche à butée dans le trou traversant axial 2, celle-ci étant alors suivie des opérations consistant à :
- percer la rotule 1 de deux trous traversants transversaux parallèles au plan P de la trochlée 11 à l'aide du gabarit 220 de perçage,
- introduire dans lesdits trous traversants transversaux les broches 201, 202,
- dégager à nouveau la palette rotulienne 22 par la mise en oeuvre du mouvement axial desdits moyens 24, 25 de déplacement,
- luxer la rotule 1 équipée des deux broches 201, 202,
- engager sur lesdites broches la platine 200 de coupe,
- couper la rotule 1 à l'aide d'une scie guidée par ladite platine de coupe.

## Revendications

1. Dispositif de coupe rotulienne pour la pose d'une prothèse totale de genou, ledit dispositif comportant, d'une part, un composant fémoral (10) d'essai présentant une trochlée prothétique (11), au moins un orifice latéral (12) aménagé sur au moins un bord dudit composant fémoral d'essai, et un trou central (13) aménagé au centre de ladite trochlée, et, d'autre part, un ancillaire (20;20') comprenant un téton (21) de fixation destiné à coopérer avec ledit orifice latéral (12) du composant fémoral (10) d'essai, une palette rotulienne (22) prévue pour être mise en appui contre la rotule (1), un moyen (23) de centrage de ladite palette par rapport audit trou central (13) de la trochlée prothétique (11), des moyens (24, 25) de déplacement de la palette rotulienne (22) permettant un mouvement transversal à ladite trochlée (11) et un mouvement axial parallèle au trou central (13), des moyens (26, 27) de blocage en position de ladite palette (22), et des moyens (100, 200) de coupe de la rotule (1) parallèlement au plan (P) de ladite trochlée (11).

2. Dispositif selon la revendication 1, caractérisé en ce que lesdits moyens de coupe sont constitués par une fente latérale (100) parallèle au plan de la trochlée prothétique (11), aménagée dans un premier gabarit (101) disposé sur l'ancillaire (20), et dans laquelle est introduite une scie oscillante.

3. Dispositif selon la revendication 1, caractérisé en ce que lesdits moyens de coupe sont constitués par une platine (200) de coupe engagée sur deux broches (201, 202) traversant la rotule (1) et mises en place parallèlement au plan de la trochlée prothétique (11) à partir de deux trous (211, 212) de passage aménagés dans un second gabarit (220) disposé sur l'ancillaire (20').

4. Dispositif selon la revendication 3, caractérisé en ce que ladite platine (200) de coupe est dissymétrique de manière à présenter deux niveaux de coupe possibles.

5. Dispositif selon l'une quelconque des revendications 1 à 4, caractérisé en ce que ledit moyen de centrage est un trou de centrage (23) aménagé au centre de ladite palette rotulienne (22) et destiné à être amené en coïncidence avec le trou central (13) du composant fémoral (10) d'essai par la mise en oeuvre dudit mouvement transversal des moyens de déplacement de ladite palette.

6. Dispositif selon l'une quelconque des revendications 1 à 5, caractérisé en ce que lesdits moyens de déplacement sont constitués par une tige transversale (24) supportant, coulissante, la palette rotulienne (22), et coulissant le long d'une tige axiale (25) fixe, parallèle au trou central (13) du composant fémoral (10) d'essai.

7. Dispositif selon la revendication 6, caractérisé en ce que lesdits moyens de blocage sont constitués par une première vis (26) de blocage de la palette rotulienne (22) sur ladite tige transversale (24) et par une deuxième vis (27) de blocage de la tige transversale sur ladite tige axiale (25).

## Claims

1. A device for cutting the patella for installing a total knee prosthesis, said device comprising on the one hand a test femur component (10) having a prosthetic trochlea (11), at least one lateral orifice (12) provided on at least one edge of the said test femur component, and a central hole (13) provided at the center of the said trochlea, and on the other hand an ancillary means (20; 20') comprising a locating spur (21) adapted for cooperation with the said lateral orifice (12) of the test femur component (10), a patellar plate (22) provided to be placed to boar against the patella (1), a means (23) for the centering of the said plate in relation to the said central hole (13) of the prosthetic trochlea (11), means (24, 25) for the displacement of the patellar plate (22) to allow a movement transverse in relation to the trochlea (11) and an axial movement parallel to the central hole (13), means (26, 27) for locking the position of the said plate (22), and means (100, 200) for cutting the patella (1) in parallelism to the plane (P) of the said trochlea (11).

2. The device as claimed in claim 1, characterized in that the said cutting means are constituted by a lateral gap (100) parallel to the plane of the prosthetic trochlea (11), provided in a first template (101), placed on the ancillary means (20) and into which a reciprocating saw is introduced.

3. The device as claimed in claim 1, characterized in that the said cutting means are constituted by a cutting blade (200) fitted on two arbors (201, 202) extending through the patella (1) and arranged in parallelism to the plane of the prosthetic trochlea (11) starting from two through holes (211, 212) provided in a second template (220) arranged on the ancillary means (20').

4. The device as claimed in claim 3, characterized in that the said cutting blade (200) is asymmetrical in such a fashion as to present two levels of possible out.

5. The device as claimed in any one of the claims 1 through 4, characterized in that the said centering means is a centering hole (23) provided at the center of the said patellar plate (22) and adapted to be brought into a position coinciding with the central hole (13) of the test femur component (10) by causing the said transverse movement of the displacement means for the said plate.

6. The device as claimed in any one of the claims 1 through 5, characterized in that the said displacement means are constituted by a transverse rod (24) for sliding and supporting, the patellar plate (22) and sliding along a fixed axial rod (25) parallel to the central hole (13) of the test femur component (10).

7. The device as claimed in claim 6, characterized in that the said locking means are constituted by a first locking screw (26) for the patellar plate (22) on the said transverse rod (24) and by a second screw (27) for locking the transverse rod on the said axial rod (25).

## Patentansprüche

1. Kniescheibenresektionsvorrichtung zum Einsetzen einer Gesamtknieprothese, die einerseits ein Probeschenkelteil (10) mit einer Prothesentrochlea (11), wenigstens eine seitliche Öffnung (12), die an wenigstens einem Rand des Probeschenkslteils vorgesehen ist, sowie ein zentrales Loch (13), das im Zentrum der Trochlea angeordnet ist, und andererseits ein Hilfselement (20; 20') mit einem Befestigungsansatz (21), der zum Zusammenwirken mit der seitlichen Öffnung (12) des Probeschenkelteils (10) bestimmt ist, eine Kniescheibenplatte (22), die zur Anlage gegen die Kniescheibe (1) vorgesehen ist, ein Mittel (23) zum Zentrieren der Platte bezüglich des zentralen Lochs (13) der Prothesentrochlea (11), Mittel (24, 25) zum Verschieben der Kniescheibe (22), die eine Querbewegung zu der Trochlea (11) sowie eine zu dem zentralen Loch (13) parallele Axialbewegung ermöglichen, Mittel (26, 27) zum Blockieren der Platte (22) an ihrer Position sowie Mittel (100, 200) zur Resektion der Kniescheibe (1) parallel zu der Ebene (P) der Trochlea (11) aufweist.

2. Vorrichtung nach Anspruch 1, dadurch gekennzeichnet, daß die Resektionsmittel durch einen parallel zur Ebene der Prothesentrochlea (11) vorgesehenen, seitlichen Schlitz (100) gebildet sind, der in einer an dem Hilfselement (20) angeordneten ersten Schablone (101) vorgesehen ist und in den eine Kappsäge eingeführt ist.

3. Vorrichtung nach Anspruch 1, dadurch gekennzeichnet, daß die Resektionsmittel durch eine Resektionsscheibe (200) gebildet sind, die an zwei Stiften (201, 202) in Eingriff gebracht sind, die die Kniescheibe (1) durchqueren und ausgehend von zwei Durchgangslöchern (211, 212), die in einer an dem Hilfselement (20') vorgesehenen zweiten Schablone (220) vorgesehen sind, parallel zu der Ebene der Prothesentrochlea (11) eingesetzt sind.

4. Vorrichtung nach Anspruch 3, dadurch gekennzeichnet, daß die Resektionsplatte (200) derart asymmetrisch ist, daß zwei Resektionsniveaus möglich werden.

5. Vorrichtung nach einem der Ansprüche 1 bis 4, dadurch gekennzeichnet, daß das Zentriermittel ein Zentrierloch (23) ist, das im Zentrum der Kniescheibe (22) vorgesehen und dazu bestimmt ist, durch die Anwendung der Querbewegung der Mittel zum Verschieben der Platte in Deckung mit dem zentralen Loch (13) des Probeschenkelteils (10) gebracht zu werden.

6. Vorrichtung nach einem der Ansprüche 1 bis 5, dadurch gekennzeichnet, daß die Verschiebungsmittel durch einen Querschaft (24) gebildet sind, der die Kniescheibe (22) gleitend stützt und entlang eines festen axialen Schaftes (25) gleitet, der parallel zu dem zentralen Loch (13) des Probeschenkelteils (10) liegt.

7. Vorrrichtung nach Anspruch 6, dadurch gekennzeichnet, daß die Blockiermittel durch eine erste Schraube (26) zum Blockieren der Kniescheibe (22) an dem Querschaft (24) sowie durch eine zweite Schraube (27) zum Blockieren des Querschafts an dem axialen Schaft (25) gebildet sind.
